# EUROPÄISCHE PATENTSCHRIFT

(11) **EP 1 259 483 B1**
(45) Veröffentlichungstag und Bekanntmachung des Hinweises auf die Patenterteilung: **16.04.2008**
(21) Anmeldenummer: 01936057.7
(22) Anmeldetag: 02.03.2001
(51) Int. Cl.: C07D 207/337, C07C 255/19

(54) **VERFAHREN ZUR HERSTELLUNG VON BETA-ALANINAMIDEN**
PROCESS FOR THE PREPARATION OF BETA-ALANINAMIDES
PROCEDE DE PREPARATION DE BETA-ALANINAMIDES

(30) Priorität: 03.03.2000 EP 00104643; 28.02.2001 US 271694 P
(43) Veröffentlichungstag der Anmeldung: 27.11.2002
(73) Patentinhaber: Lonza AG, 4052 Basel (CH)
(72) Erfinder: HILDBRAND, Stefan, CH-4125 Riehen (CH); RUPPEN, Thomas, CH-3904 Naters (CH); VEGHINI, Dario, CH-3900 Brig (CH)
(86) Internationale Anmeldenummer: PCT/EP2001/002349
(87) Internationale Veröffentlichungsnummer: WO 2001/064638

(56) Entgegenhaltungen:
- EP-A- 0 294 668
- WO-A-91/05555
- GB-A- 1 500 576
- US-A- 4 359 416
- US-A- 5 112 751
- R O C NORMAN: "Principles of Organic Synthesis" 1968 , METHUEN & CO LTD / SCIENCE PAPERBACKS , LONDON XP002172715 Seite 572

## Beschreibung

Die Erfindung betrifft ein Verfahren zur Herstellung von Amiden des β-Alanins, insbesondere von Dipeptiden wie beispielsweise Carnosin. Sie betrifft weiterhin neue Cyanessigsäureamide als Zwischenprodukte im erfindungsgemässen Verfahren.

Carnosin (β-Alanyl-L-histidin) ist ein natürlich vorkommendes Dipeptid der nachstehenden Struktur, welches aus Muskelgewebe isoliert werden kann. Carnosin ist als potentielles Therapeutikum und neuerdings auch als Nahrungsmittelzusatz (Dietary Supplement) mit antioxidativer Wirkung von Interesse.

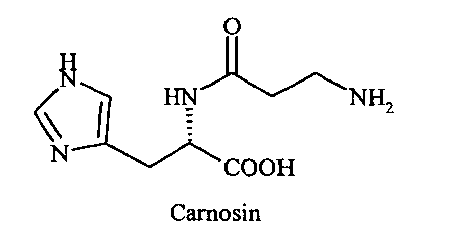

Es sind bereits mehrere Synthesen von Camosin aus den Bestandteilen L-Histidin und β-Alanin bzw. entsprechenden Derivaten bekannt, welche jedoch den Einsatz von Schutzgruppen und/oder aktivierten Derivaten erfordern und deshalb für die kostengünstige Herstellung grosser Mengen wenig geeignet sind. So beschreibt z. B. US-A-4 359 416 die Herstellung von Camosin aus L-Histidin und dem aus β-Alanin erhältlichen Dihydro-1,3-thiazin-2,6-dion. EP-A-0 294 668 beschreibt (2-Cyan-2-oximinoacetyl)-aminosäure-Derivate. Offenbart wird darin die Umsetzung von Aminosäureester-Hydrochloriden mit Cyanessigsäuremethylester zu *N*-Cyanacetylaminosäureestern.

Aufgabe der vorliegenden Erfindung war daher, ein für die technische Synthese von Camosin und anderen Dipeptiden des β-Alanins geeignetes Verfahren bereitzustellen, das ohne die Verwendung von Schutzgruppen oder teuren Derivaten auskommt und nur gut zugängliche Ausgangsmaterialien einsetzt.
Erfindungsgemäss wird diese Aufgabe durch das Verfahren nach Patentanspruch 1 gelöst. Die erfindungsgemäss herstellbaren β-Alaninamide besitzen die allgemeine Formel

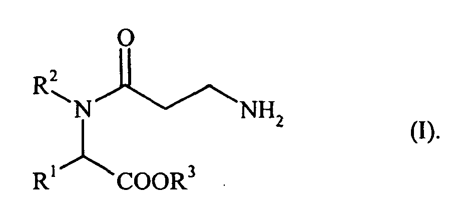

Hierin bedeutet R¹ Wasserstoff oder ein gegebenenfalls mit Hydroxy, Amino, Carboxy, Carbamoyl, Methylmercapto, Guanidino, Aryl oder Heteroaryl, wobei Aryl oder Heteroaryl seinerseits mit den zuvor genannten Gruppen substituiert sein kann, substituiertes C₁₋₆-Alkyl und R² ist Wasserstoff, oder R¹ und R² bilden zusammen eine Gruppe der Formel -(CH₂)*ₙ*-, worin *n* 3 oder 4 ist. R³ ist Wasserstoff, eine durch ein Äquivalent eines anorganischen oder organischen Kations kompensierte negative Ladung oder C₁₋₆-Alkyl. Diese Verbindungen könnnen in neutraler Form oder nach Deprotonierung der Carboxylgruppe (R³ = negative Ladung) und/oder Protonierung der primären Aminogruppe als innere Salze oder Salze mit Basen bzw. Säuren vorliegen. Einige der Verbindungen, insbesondere solche, die Imidazolylreste enthalten, können auch ich mehreren tautomeren Formen oder als Gemisch solcher Formen vorliegen. Wenn R¹ von Wasserstoff verschieden ist, enthalten die β-Alaninamide (I) wenigstens ein Asymmetriezentrum. Die Formel I umfasst hierbei sowohl alle jeweils möglichen Stereoisomeren als auch deren Gemische.

Unter C₁₋₆-Alkyl sind hier und im folgenden alle linearen oder verzweigten primären, sekundären oder tertiären Alkylgruppen mit 1 bis 6 Kohlenstoffatomen zu verstehen, also beispielsweise Methyl, Ethyl, Propyl, Isopropyl, Butyl, *sec-*Butyl*, tert*-Butyl, Isobutyl, Pentyl, Isopentyl, Neopentyl, 2-Methylbutyl, Hexyl, 2-Methylpentyl, 3-Methylpentyl usw. Entsprechendes gilt für C₁₋₁₀-Alkyl, wozu neben den bereits genannten beispielsweise noch Gruppen wie Octyl oder 2-Ethylhexyl zählen.

Unter Aryl sind mono- oder polycyclische carbocyclische aromatische Gruppen wie insbesondere Phenyl oder Naphthyl zu verstehen, unter Heteroaryl entsprechend mono- oder polycyclische heterocyclische aromatische Gruppen mit einem oder mehreren Heteroatomen, insbesondere Imidazolyl oder Indolyl. Arylgruppen können gegebenfalls einen oder mehrere der oben erwähnten Substituenten tragen, insbesondere Hydroxygruppen wie beispielsweise in 4-Hydroxyphenyl.

Die gegebenenfalls (wenn R³ = negative Ladung) vorhandenen anorganischen Kationen können ein- oder mehrwertig sein. Einwertige anorganische Kationen sind beispielsweise die Alkalimetallionen wie Li⁺, Na⁺, K⁺, Rb⁺ und Cs⁺. Mehrwertige anorganische Kationen sind beispielsweise die Erdalkalimetallionen wie Mg²⁺, Ca²⁺, Sr²⁺ und Ba²⁺. Organische Kationen sind beispielsweise quartäre Ammoniumionen.

Es wurde gefunden, dass α-Aminosäuren, deren Ester und Salze der allgemeinen Formel 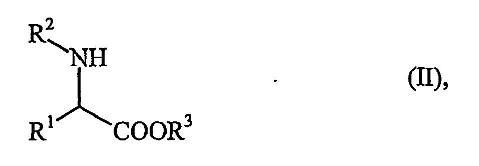 worin R¹, R² und R³ die oben genannten Bedeutungen haben, mit einem Cyanessigsäureester der allgemeinen Formel 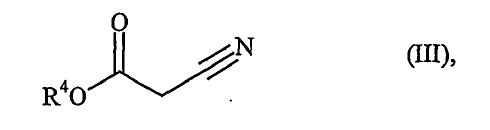 worin R⁴ C₁₋₁₀-Alkyl ist, zu einem Cyanessigsäureamid der allgemeinen Formel 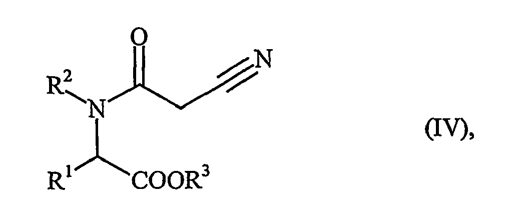 worin R¹, R² und R³ die oben genannten Bedeutungen haben, oder einem entsprechenden Salz umgesetzt werden können und das Cyanessigsäureamid (IV) durch katalytische Hydrierung in die Zielverbindung (I) oder ein entsprechendes Salz übergeführt werden kann.

Vorzugsweise ist R¹ Wasserstoff oder gegebenenfalls substituiertes C₁₋₄-Alkyl, insbesondere Methyl, Isopropyl, Isobutyl, *sec*-Butyl, Indol-3-ylmethyl, Benzyl, *p*-Hydroxybenzyl, 2-(Methylsulfanyl)ethyl, Hydroxymethyl, 1-Hydroxyethyl, Carbamoylmethyl, 2-Carbamoylethyl, Carboxymethyl, 2-Carboxyethyl, 4-Aminobutyl oder 3-Guanidinopropyl.

R² ist vorzugsweise Wasserstoff.

Besonders bevorzugt ist R¹ Imidazol-4-ylmethyl, das gegebenenfalls wie oben angegeben substituiert sein kann, und R² ist Wasserstoff.

R⁴ ist vorzugsweise Methyl oder Ethyl.

Falls die α-Aminosäure bzw. deren Ester (II) als inneres Salz bzw. Salz vorliegt, in dem die α-ständige Aminogruppe protoniert ist, muss diese zunächst durch Zugabe einer Base deprotoniert werden. Als Base sind hierbei grundsätzlich alle Basen verwendbar, die eine stärkere Basizität als die α-Aminogruppe der Aminosäure (II) aufweisen. Vorzugsweise wird eine mittelstarke bis starke Base eingesetzt. Hierfür eignen sich beispielsweise Alkalihydroxide wie Natrium- oder Kaliumhydroxid, tertiäre Amine wie Triethylamin, 4-Dimethylaminopyridin, 1,4-Diaza[2.2.2]bicyclooctan, bicyclische Amidine ("DBN", "DBU") sowie in nichtwässrigen Lösungsmitteln Alkalialkoholate wie Natriummethanolat oder -ethanolat und in aprotischen Lösungsmitteln auch Alkalimetallhydride und -amide wie beispielsweise Natriumhydrid oder -amid. Die Base wird vorzugsweise in stöchiometrischer oder annähernd stöchiometrischer Menge eingesetzt.

Bevorzugte Lösungsmittel für die erste Stufe sind polare protische oder aprotische Lösungsmittel wie Wasser, C₁₋₄-Alkanole wie beispielsweise Methanol oder Ethanol, und Amide wie beispielsweise *N-N*-Dimethylformamid, *N,N-*Dimethylacetamid, 1-Methyl-2-pyrrolidon oder 1,3-Dimethyl-3,4,5,6-tetrahydro-2(1*H*-pyrimidinon (DMPU).

Als Katalysatoren für die Hydrierung werden vorzugsweise Metallkatalysatoren auf Basis von Nickel, Cobalt, Kupfer, Rhodium, Palladium, Ruthenium oder Platin eingesetzt, die gegebenenfalls auf einem Träger aufgezogen sind. Hierzu zählen beispielsweise Nickel- und Cobaltkatalysatoren vom Raney-Typ, feinverteiltes Platin (z.B. durch Reduktion von PtO₂ erhalten), Rhodium, Palladium oder Platin auf Aktivkohle oder Aluminiumoxid oder Cobalt auf Siliciumdioxid (Silica).

Besonders bevorzugt sind Raney-Nickel und Raney-Cobalt sowie Rhodium auf Aktivkohle oder Aluminiumoxid.
Als Lösungsmittel in der Hydrierung können die bei der Hydrierung von Nitrilen zu Aminen üblichen Lösungsmittel wie beispielsweise Wasser, konzentrierte wässrige Ammoniaklösung, Methanol, Ethanol, *N,N*-Dimethylformamid oder Gemische der genannten Lösungsmittel eingesetzt werden.

Die Cyanessigsäureamide der allgemeinen Formel 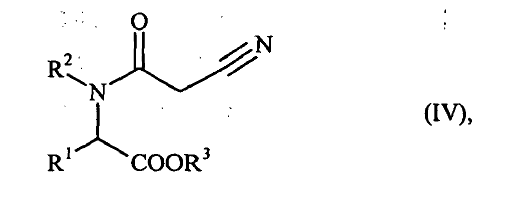 worin R¹ Imidazol-4-ylmethyl, das gegebenenfalls wie oben für Formel I beschrieben substituiert ist, und R² Wasserstoff ist und R³ die oben genannte Bedeutung hat, sowie deren Salze sind neu und ebenfalls Gegenstand der Erfindung. Als Substituenten kommen hierbei beispielsweise C₁₋₆-Alkylgruppen in Frage, insbesondere Methyl.

Besonders bevorzugte Cyanessigsäureamide (IV) sind *N*^{α}-(Cyanacetyl)-L-histidin und dessen Ester der Formel 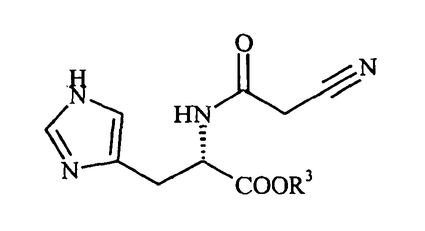 worin R³ die oben genannte Bedeutung hat, und deren Salze und Tautomere sowie *N*^{α}-(Cyanacetyl)-3-methyl-L-histidin und dessen Ester der Formel 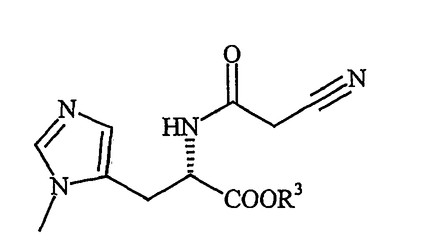 worin R³ die oben genannte Bedeutung hat, sowie deren Salze.

Die folgenden Beispiele verdeutlichen die Durchführung des erfindungsgemässen Verfahrens und die Herstellung der erfindungsgemässen Verbindungen.

### Beispiel 1

### (S)-2-(Cyanacetylamino)-3-(1H-imidazol-4-yl)propionsäure-Natriumsalz

Zu einer durch Auflösen von 5,57 g (0,24 mol) Natrium in 800 ml Ethanol erhaltenen Lösung von Natriumethanolat wurden bei Raumtemperatur 40,0 g (0,26 mol) L-Histidin gegeben. Nach 15 min wurden 44,12 g (0,39 mol) Cyanessigsäureethylester zugegeben und die Suspension 16 h am Rückfluss gekocht. Nach dem Abkühlen auf Raumtemperatur wurde das Gemisch filtriert. Das gelbliche Filtrat wurde im Vakuum eingeengt, der Rückstand in Ethylacetat aufgeschlämmt, filtriert, mit Ethylacetat gewaschen und mittels FlashChromatographie an Kieselgel (Elutionsmittel: Gradient Ethylacetat → Methanol/Ethylacetat 3:1) gereinigt.
Ausbeute: 28,42 g (46%)
¹H NMR (DMSO-*d*₆, 400 MHz): δ = 8,28 (d, 1H); 7,45 (s, 1H); 6,7 (s, 1H); 5,5 (br. s, 1H); 4,12-4,20 (m, 1H); 3,65 (s, 2H); 2,95-3,05 (m, 1H); 2,8-2,9 (m, 1H).
¹³C NMR (DMSO-*d*₆, 100 MHz): δ = 174,05; 161,09; 134,25; 131,97; 119,66; 116,43; 54,83; 29,13; 25,20.

### Beispiel 2

### (S)-2-(Cyanacetylamino)-3-(1H-imidazol-4-yl)propionsäure-Natriumsalz

Zu einer Suspension von 40,0 g (0,26 mol) L-Histidin in 750 ml *N,N*-Dimethylformamid wurden bei Raumtemperatur 9,80 g Natriumhydrid (60% in Mineralöl) und 50,6 g (0,51 mol) Cyanessigsäuremethylester gegeben. Das Gemisch wurde im offenen Kolben 2 h auf 155°C erhitzt und die so erhaltene Lösung mittels HPLC analysiert. Identifiziert wuden Histidin (8 Flächen-%) und (*S*)-2-(Cyanacetylamino)-3-(1*H*-imidazol-4-yl)propionsäure-Natriumsalz (38 Flächen-%).

### Beispiel 3

### (S)-2-(Cyanacetylamino)-3-(1H-imidazol-4-yl)propionsäure

Zu einer durch Auflösen von 4,02 g (0,175 mol) Natrium in 280 ml Ethanol erhaltenen Lösung von Natriumethanolat wurden bei Raumtemperatur 28,27 g (0,18 mol) L-Histidin gegeben. Das Gemisch wurde langsam aufgeheizt und bei einer Temperatur von 60 °C wurden 30,92 g (0,27 mol) Cyanessigsäureethylester zugetropft. Das Gemisch wurde weiter aufgeheizt und das Ethanol wurde abdestilliert, wobei die abdestillierte Ethanolmenge laufend portionsweise durch *N,N*-Dimethylformamid ersetzt wurde. Am Schluss der Reaktion war die Temperatur der Lösung 130°C. Bei dieser Temperatur wurde weitere 2 h gerührt. Das braune Reaktionsgemisch (200 g) wurde auf 50 °C abgekühlt und 30 g konzentrierte Salzsäure wurden zudosiert. Unter vermindertem Druck wurden dann ca. 70 g Lösungsmittel (H₂O/*N,N*-Dimethylformamid-Gemisch) abdestilliert. Die dickflüssige Suspension wurde mit 200 g Aceton versetzt, auf -10°C abgekühlt und filtriert. Zur Umkristallisation wurde der Rückstand in Wasser aufgelöst und der pH wurde auf 5,0 eingestellt. Beim Abkühlen (<5 °C) fiel ein weisser Feststoff aus, der abfiltriert, mit Ethanol gewaschen und bei 40 °C/20 mbar getrocknet wurde.
Ausbeute: 26,39 g (66%).
IR (KBr): ν̃ = 3421, 3240, 3149, 3059, 2970, 2255, 1653, 1551, 1396, 1107, 1088, 979, 965, 826, 786, 638 cm⁻¹.
¹H NMR (DMSO-*d₆*, 400 MHz): δ =11,0 (br., 2H); 8,50 (d, 1H); 7,68 (s, 1H); 6,85 (s, 1H); 4,35-4,48 (m, 1H); 3,68 (s, 2H); 2,92-3,03 (m, 1H); 2,82-2,91 (m, 1H).
¹³C NMR (DMSO-*d₆*, 100 MHz): δ = 172,23; 161,92; 134,55; 132,70; 116,73; 115,87; 52,80; 28,68; 25,06.
LC-MS: *m*/*z* = 223 ([M+H]⁺), 205, 177, 156, 110.

An einem gemäss vorstehender Vorschrift erhaltenen Muster wurde die optische Reinheit zu >99,8% bestimmt. Die Bestimmung erfolgte durch Hydrolyse der Amidbindung (6 N Salzsäure, 110 °C, 24 h), gefolgt von einer Derivatisierung des freigesetzten Histidins mit Trifluoressigsäureanhydrid und Chlorameisensäureisobutylester. Mittels Gaschromatographie an einer chiralen stationären Phase wurde ein Gehalt an D-Histidin von <0,1 % nachgewiesen.

### Beispiel 4

### L-Carnosin

Zu einer Lösung von 1,90 g (7,8 mmol) (*S*)-2-(Cyanacetylamino)-3-(1*H*-imidazol-4-yl)-propionsäure-Natriumsalz (hergestellt gemäss Beispiel 1) in 50 ml Ethanol/konz. Ammoniaklösung (*V:V*= 4:1) wurden 0,3 g Rhodium/Aktivkohle (5% Rh) gegeben. Das Gemisch wurde bei 110 °C und 45 bar 1 h hydriert. Anschliessend wurde der Katalysator abfiltriert und das Filtrat mit Ameisensäure auf pH 8,2 gestellt. Nach dem Einengen der Lösung im Vakuum wurde der Rückstand in 200 ml Ethanol aufgeschlämmt und 30 min auf 60 °C erwärmt. Das Produkt wurde abfiltriert, nacheinander mit Ethanol, Ethylacetat und Diethylether gewaschen und schliesslich getrocknet.
Ausbeute: 1,33 g (76%)
¹H NMR (D₂O, 400 MHz): δ = 7,70 (s, 1H); 6,93 (s, 1H); 4,43-4,50 (m, 1H); 3,20-3,28 (m, 2H); 3,11-3,19 (m, 1H); 2,95-3,03 (m, 1H); 2,61-2,71 (m, 2H).

Die optische Reinheit wurde nach der in Beispiel 3 beschriebenen Methode zu 99,5% bestimmt.

### Beispiel 5

### (S)-2-(Cyanacetylamino)-3-(1H-imidazol-4-yl)propionsäuremethylester

Zu einer durch Auflösen von 0,94g (40,7 mmol; 1,95 Äquiv.) Natrium in 100 ml Methanol erhaltenen Lösung von Natriummethanolat wurden bei Raumtemperatur 5,0 g (20,4 mmol) L-Histidinmethylester-Dihydrochlorid zugegeben. Nach 30 min wurden 3,03 g (30,6 mmol) Cyanessigsäuremethylester zugegeben und das Gemisch wurde 16 h am Rückfluss gekocht. Nach dem Abkühlen auf Raumtemperatur wurde das Gemisch filtriert. Das gelbliche Filtrat wurde im Vakuum eingeengt und der Rückstand mittels FlashChromatographie an Kieselgel (Elutionsmittel: Gradient Ethylacetat → Ethylacetat/Methanol 3:1) gereinigt.
Ausbeute: 1,51g (31%)
¹H NMK (DMSO-*d₆*, 400 MHz): δ = 8,65 (d, 1H); 7,52 (s, 1H); 6,8 (s, 1H); 4,45-4,55 (m, 1H); 3,69 (s, 2H); 3,62 (s, 3H); 3,3 (br., 1H); 2,82-2,98 (m, 2H).

### Beispiel 6

### L-Carnosin

In einem 1 Liter Druckautoklaven wurden 1,76 g Rh/C (0,4 mol% reines Rh bezüglich eingesetztem Edukt) in einem Gemisch aus 94,2 g Ammoniaklösung (25% in H₂O) und 62,8 g Methanol vorgelegt. Der Autoklav wurde geschlossen, der Inhalt wurde auf 90 °C aufgeheizt und 40 bar Wasserstoff wurden aufgepresst. Innerhalb einer Stunde wurde dann eine Lösung von 20,0 g (0,09 mol) (*S*)-2-(Cyanacetylamino)-3-(1*H*-imidazol-4-yl)-propionsäure (hergestellt gemäss Beispiel 3) in einem Gemisch 94,2 g Ammoniaklösung (25% in H₂O) und 62,8 g Methanol zudosiert. Nach einer einstündigen Nachreaktion bei 90 °C wurde das Reaktionsgemisch auf Raumtemperatur abgekühlt. Der Autoklav wurde entspannt und der Katalysator über Aktivkohle abfiltriert. Eine HPLC Inprozess-Analyse zeigte, dass die klare grünliche Reaktionslösung (326,2 g) 5,74% (*m*/*m*) Carnosin enthielt, was bei vollständigem Umsatz einer Selektivität von 92% entspricht. Das Reaktionsgemisch wurde anschliessend am Rotationsverdampfer auf ca. 60 g aufkonzentriert. Durch die tropfenweise Zugabe von 174 g Ethanol fiel ein weisser Feststoff aus, der abfiltriert und bei 50 °C/20 mbar getrocknet wurde.

Ausbeute: 13,0 g (64%)
¹H NMR (D₂O, 400 MHz): δ = 7,70 (s, 1H); 6,93 (s, 1H); 4,43-4,50 (m, 1H); 3,20-3,28 (m, 2H); 3,11-3,19 (m, 1H); 2,95-3,03 (m, 1H); 2,61-2,71 (m, 2H).
¹³C NMR (D₂O, 100 MHz): δ = 178,58; 172,39; 136,46; 133,90;118,37; 55,99; 36,65; 33,09; 29,74.
LC-MS: *m*/*z* = 227 ([M+H]⁺), 210, 192, 164, 146, 136, 110.

### Beispiel 7

### L-Carnosin

In einem 1 Liter Druckautoklaven wurden zu 0,88 g Rh/C (0,4 mol% reines Rh bezüglich eingesetztem Edukt) eine Lösung von 10,00 g (45,0 mmol) (*S*)-2-(Cyanacetylamino)-3-(1*H*-imidazol-4-yl)propionsäure (hergestellt gemäss Beispiel 3) in einem Gemisch von 157 g konz. NH₃/Methanol (m/m = 3:2) zugegeben. Der Autoklav wurde geschlossen und zweimal mit 40 bar Stickstoff und einmal mit Wasserstoff gespült. Das Gemisch wurde auf 90 °C geheizt und 40 bar Wasserstoff wurden aufgepresst. Nach 3 h bei 90 °C wurde das Reaktionsgemisch auf Raumtemperatur abgekühlt, der Autoklav entspannt und der Katalysator durch Filtration abgetrennt. Eine Inprozess-Analyse (HPLC) zeigte, dass die Reaktionslösung (147,2 g) 6,38% (*m*/*m*) Camosin enthielt, was bei vollständigem Umsatz einer Selektivität von 92% entspricht. Das Reaktionsgemisch wurde anschliessend am Rotationsverdampfer auf 41,2 g aufkonzentriert. Bei Raumtemperatur wurden 124 g Ethanol zugetropft und der Kolben wurde über Nacht in einen Kühlschrank gestellt. Am nächsten Tag wurde der Niederschlag abfiltriert, mit Ethanol gewaschen und im Trockenschrank bei 40 °C/20 mbar getrocknet. Es wurden 7,96 g (78%) eines leicht grünlichen Feststoffs mit einem Gehalt (HPLC) von 98,0% (*m*/*m*) erhalten.

### Beispiel 8

### L-Carnosin

Es wurde verfahren wie in Beispiel 7 beschrieben, mit dem Unterschied dass 5% Rh auf Aluminiumoxid als Katalysator eingesetzt wurde. Unter diesen Bedingungen wurde L-Carnosin mit 83% Selektivität gebildet.

### Beispiel 9

### L-Carnosin

In einem 1 Liter Druckautoklaven wurden 4,5 g Raney-Cobalt (mit 0,3% Eisen dotiert) in 195 g Methanol vorgelegt. Eine Lösung von 30,0 g (0,135 mol) (*S*)-2-(Cyanacetylamino)-3-(1*H*-imidazol-4-yl)propionsäure (hergestellt gemäss Beispiel 3) in 375 g Ammoniaklösung (25% in H₂O) wurde zugeben. Der Autoklav wurde geschlossen und zweimal mit 40 bar Stickstoff gespült. Dann wurden 45 bar Wasserstoff aufgepresst und der Inhalt wurde innerhalb einer halben Stunde auf 100 °C aufgeheizt. Nach einer Nachreaktion von 3 h bei 100 °C wurde das Reaktionsgemisch auf Raumtemperatur abgekühlt und der Autoklav entspannt. Eine HPLC Inprozess-Analyse zeigte, dass die Reaktionslösung (590,8 g) 4,68 % (*m*/*m*) Carnosin enthielt, was bei vollständigem Umsatz einer Selektivität von 91% entspricht.

### Beispiel 10

### L-Carnosin

In einem 100 ml Druckautoklaven wurden zu einer Lösung von 2,0 g (9,0 mmol) (*S*)-2-(Cyanacetylamino)-3-(1*H*-imidazol-4-yl)propionsäure (hergestellt gemäss Beispiel 3) in einem Gemisch von 25 g Ammoniaklösung (25% in H₂O) und 13 g Methanol 1,1 g Raney-Nickel (mit 1,8% Molybdän dotiert) zugegeben. Der Autoklav wurde geschlossen und in ein auf 100 °C vorgeheiztes Ölbad gestellt. Nach 10 Minuten wurden 50 bar Wasserstoff aufgepresst. Nach 2,5 h bei 100 °C wurde das Reaktionsgemisch auf Raumtemperatur abgekühlt und der Autoklav entspannt. Eine HPLC Inprozess-Analyse zeigte, dass die Reaktionslösung (39,4 g) 4,54 % (*m*/*m*) Carnosin enthielt, was bei einem Umsatz von 99% einer Selektivität von 89% entspricht.

### Beispiel 11

### L-Carnosin

In einem 1 Liter Druckautoklaven wurden 4,50 g Raney-Cobalt (mit 0,3% Eisen dotiert) in einem Gemisch von 285 g konz. Ammoniak/Methanol (*m*/*m* = 1,9:1) vorgelegt. Der Autoklav wurde geschlossen und zweimal mit 40 bar Stickstoff gespült. Dann wurden 45 bar Wasserstoff aufgepresst und das Gemisch wurde auf 100 °C aufgeheizt. Innerhalb einer Stunde wurde dann eine Lösung von 30,0 g (0,135 mol) (*S*)-2-(Cyanacetylamino)-3-(1*H-*imidazol-4-yl)propionsäure (hergestellt gemäss Beispiel 3) in einem Gemisch von 285 g konz. Ammoniak/Methanol (*m*/*m* = 1,9:1) zudosiert. Nach einer einstündigen Nachreaktion bei 100 °C wurde das Reaktionsgemisch auf Raumtemperatur abgekühlt. Der Autoklav wurde entspannt und der Katalysator abfiltriert. Eine HPLC Inprozess-Analyse zeigte, dass die rötlich braune Reaktionslösung (310,5 g) 9,57% (*m*/*m*) Carnosin enthielt, was bei vollständigem Umsatz einer Selektivität von 97% entspricht.

### Beispiel 12

### (S)-2-(Cyanacetylamino)-3-(3-methyl-3H-imidazol-4-yl)propionsäure-Natriumsalz

Zu einer Lösung von 0,20 g (2,94 mmol) Natriumethanolat in 5,60 g Ethanol wurden bei 40 °C 0,50 g (2,95 mmol) 3-Methyl-L-histidin zugegeben. Die klare Lösung wurde auf 60 °C geheizt und 0,50 g (4,43 mmol) Cyanessigsäureethylester wurden tropfenweise zugegeben. Das Gemisch wurde 1 h am Rückfluss erhitzt. Anschliessend wurden 10 mg (0,15 mmol) Imidazol zugegeben. Dann wurde das Ethanol langsam abdestilliert und die abdestillierte Ethanolmenge wurde laufend portionsweise durch *N,N*-Dimethylformamid ersetzt. Nach einer Nachreaktionszeit von 2 h bei 125 °C wurde das Reaktionsgemisch vorsichtig aufkonzentriert und der Rückstand mittels Flash-Säulenchromatographie an Kieselgel (Elutionsmittel: Gradient Ethylacetat → Ethylacetat/Methanol 2:1) gereinigt. Es wurden 0,49 g (64%) eines leicht gelblichen Feststoffes erhalten.
DC: *R*_{f} = 0,46 (Ethanol/H₂O 3:7).
¹H NMR (DMSO-*d*₆, 400 MHz): δ = 7,91 (d, 1H); 7,38 (s, 1H); 6,58 (s, 1H); 3,97 (q, 1H); 3,68 (s, 2H); 3,50 (s, 3H); 3,01 (dd, 1H); 2,85 (dd, 1H).
¹³C NMR (DMSO-*d*₆, 100 MHz): δ =171,54; 160,80; 136,95; 128,68; 126,91; 116,40; 54,26; 30,65; 25,97; 25,11.
LC-MS: *m*/*z* = 237 ([M+H]⁺), 219, 193, 191, 176, 166, 164, 150, 109.

### Beispiel 13

### (S)-2-(3-Aminopropionylamino)-3-(3-methyl-3H-imidazol-4-yl)propionsäure (= Anserin)

Zu einer Lösung von 0,20 g (0,77 mmol) (*S*)-2-(Cyanacetylamino)-3-(3-methyl-3*H-*imidazol-4-yl)propionsäure-Natriumsalz (hergestellt gemäss Beispiel 12) in 2,4g Methanol und 1,6 g Ammoniaklösung (25% in H₂O) wurden 16 mg Rhodium/Al₂O₃ (5% Rh) zugegeben. Das Gemisch wurde bei 85 °C und 50 bar 1 h hydriert. Anschliessend wurde der Katalysator abfiltriert. Im Filtrat konnte Anserin mittels Dünnschichtchromatographie, HPLC (durch Co-Injektion mit kommerzieller Referenzsubstanz) und LC-MS eindeutig nachgewiesen werden.
Rohausbeute: ca. 45%.
DC: *R*_{f}= 0,25 (Ethylacetat/Methanol/Ammoniak/H₂O 43:35:8:10).
LC-MS: *m*/*z* = 241 ([M+H]⁺), 224, 206, 180, 170, 126, 109.

## Patentansprüche

1. Verfahren zur Herstellung von β-Alaninamiden der allgemeinen Formel 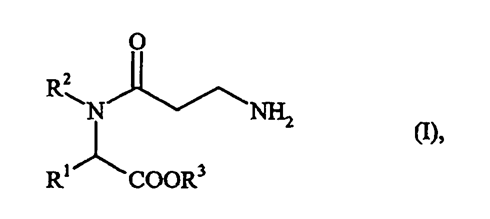 worin
(i) R¹ Wasserstoff oder gegebenenfalls mit Hydroxy, Amino, Carboxy, Carbamoyl, Methylmercapto, Guanidino oder, seinerseits gegebenenfalls mit Hydroxy, Amino, Carboxy, Carbamoyl, Methylmercapto, Guanidino oder C₁₋₆-Alkyl substituiertem, Aryl oder Heteroaryl substituiertes C₁₋₆-Alkyl bedeutet und R² Wasserstoff ist, oder
(ii) R¹ und R² zusammen eine Gruppe der Formel -(CH₂)*ₙ*- bilden, worin *n* 3 oder 4 ist, und R³ Wasserstoff, eine durch ein Äquivalent eines anorganischen oder organischen Kations kompensierte negative Ladung oder C₁₋₆-Alkyl ist,
**dadurch gekennzeichnet, dass** eine Aminosäure oder ein Aminosäureester der allgemeinen Formel 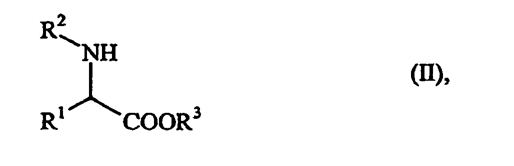 worin R¹, R² und R³ die oben genannten Bedeutungen haben, oder ein entsprechendes Salz in einer ersten Stufe mit einem Cyanessigsäureester der allgemeinen Formel 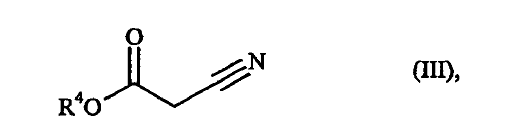 worin R⁴ C₁₋₁₀-Alkyl ist, zu einem Cyanessigsäureamid der allgemeinen Formel 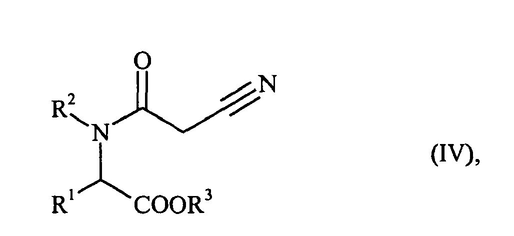 worin R¹, R² und R³ die oben genannten Bedeutungen haben, oder einem entsprechenden Salz umgesetzt wird, welches in einer zweiten Stufe durch katalytische Hydrierung in die Zielverbindung (I) oder ein entsprechendes Salz übergeführt wird.

2. Verfahren nach Anspruch 1, **dadurch gekennzeichnet, dass** R¹ Imidazol-4-ylmethyl ist, das gegebenenfalls wie in Anspruch 1 für R¹ angegeben substituiert sein kann, und R² Wasserstoff ist.

3. Verfahren nach Anspruch 1 oder 2, **dadurch gekennzeichnet, dass** als Lösungsmittel in der ersten Stufe Ethanol und/oder *N,N*-Dimethylformamid eingesetzt wird.

4. Verfahren nach einem der Ansprüche 1 bis 3, **dadurch gekennzeichnet, dass** als Katalysator in der zweiten Stufe ein Katalysator aus der Gruppe bestehend aus Rhodium auf Aktivkohle, Rhodium auf Aluminiumoxid, Raney-Nickel und Raney-Cobalt eingesetzt wird.

5. Cyanessigsäureamide der allgemeinen Formel 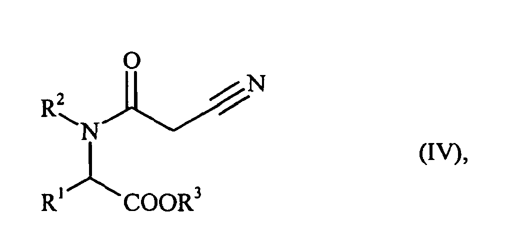 worin R¹ und R² die in Anspruch 2 genannte Bedeutung haben und R³ gemäss Anspruch 1 definiert ist, sowie deren Salze.

6. *N*^{α}-(Cyanacetyl)-L-histidin und dessen Ester der Formel 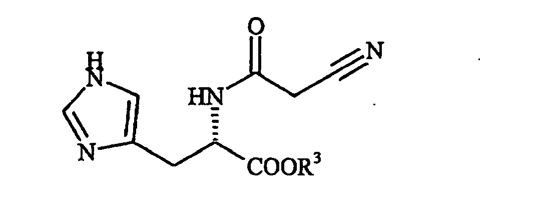 worin R³ die in Anspruch 1 genannte Bedeutung hat, sowie deren Salze und Tautomere.

7. *N*^{α}-(Cyanacetyl)-3-methyl-L-histidin und dessen Ester der Formel 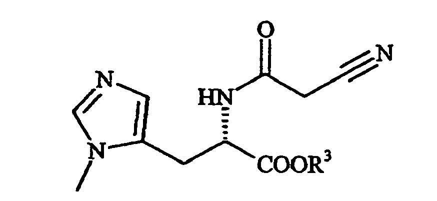 worin R³ die in Anspruch 1 genannte Bedeutung hat, sowie deren Salze.

## Claims

1. Method for preparing β-alaninamides of the general formula 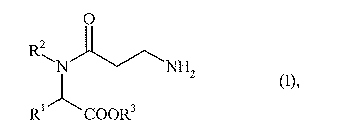 wherein
(i) R¹ is hydrogen or C₁₋₆ alkyl which is unsubstituted or substituted with hydroxy, amino, carboxy, carbamoyl, methylmercapto or guanidino, or with aryl or heteroaryl which in turn may be substituted with hydroxy, amino, carboxy, carbamoyl, methylmercapto, guanidino or C₁₋₆ alkyl, and R² is hydrogen, or
(ii) R¹ and R² together form a group of the formula -(CH₂)*ₙ*- where *n* is 3 or 4,
and R³ is hydrogen, a negative charge compensated by one equivalent of an inorganic or organic cation or is C₁₋₆ alkyl,
**characterized in that** an amino acid or an amino acid ester of the general formula 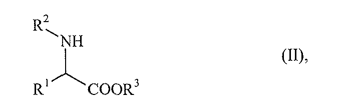 wherein R¹, R² and R³ have the abovementioned meanings, or a corresponding salt is reacted in a first stage with a cyanoacetic ester of the general formula 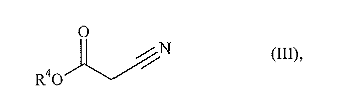 wherein R⁴ is C₁₋₁₀ alkyl to give a cyanoacetamide of the general formula 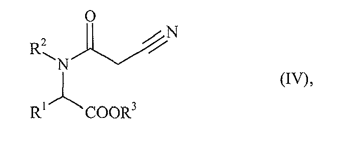 wherein R¹, R² and R³ have the abovementioned meanings or a corresponding salt, which is converted in a second stage into the target compound (I) or a corresponding salt by catalytic hydrogenation.

2. Method according to claim 1, **characterized in that** R¹ is imidazol-4-ylmethyl, which may optionally be substituted as defined in claim 1 for R¹, and R² is hydrogen.

3. Method according to claim 1 or 2, **characterized in that** the solvent used in the first stage is ethanol and/or *N,N*-dimethylformamide.

4. Method according to any one of claims 1 to 3, **characterized in that** the catalyst used in the second stage is a catalyst from the group consisting of rhodium on activated carbon, rhodium on aluminum oxide, Raney nickel and Raney cobalt.

5. Cyanoacetamides of the general formula 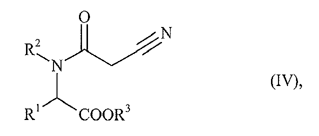 wherein R¹ and R² have the meanings mentioned in claim 2 and R³ is defined according to claim 1 and the salts thereof.

6. *N*^{α}-(Cyanoacetyl)-L-histidine and esters thereof of the formula 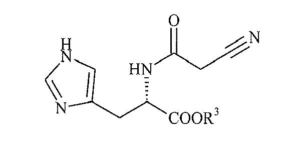 wherein R³ has the meaning mentioned in claim 1 and the salts and tautomers thereof.

7. *N*^{α}-(Cyanoacetyl)-3-methyl-L-histidine and esters thereof of the formula 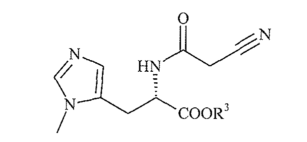 wherein R³ has the meaning mentioned in claim 1 and salts thereof.

## Revendications

1. Procédé de préparation de β-alaninamides de formule générale 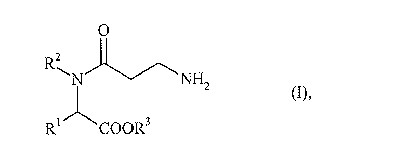 dans laquelle
(i) R¹ représente un atome d'hydrogène ou un groupe alkyle en C₁₋₆ éventuellement substitué par un groupe hydroxy, un groupe amino, un groupe carboxy, un groupe carbamoyle, un groupe méthylmercapto, un groupe guanidino ou par un groupe aryle ou un groupe hétéroaryle éventuellement substitué, de son côté, par un groupe hydroxy, un groupe amino, un groupe carboxy, un groupe carbamoyle, un groupe méthylmercapto, un groupe guanidino ou un groupe alkyle en C₁₋₆, et R² représente un atome d'hydrogène, ou
(ii) R¹ et R² forment conjointement un groupe de formule -(CH₂)*ₙ*-, dans laquelle *n* vaut 3 ou 4,
et R³ représente un atome d'hydrogène, une charge négative compensée par un équivalent d'un cation inorganique ou organique ou un groupe alkyle en C₁₋₆, **caractérisé en ce qu'**un acide aminé ou un ester d'acide aminé de formule générale 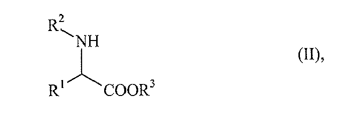 dans laquelle R¹, R² et R³ présentent les significations mentionnées ci-dessus, ou un sel correspondant est mis à réagir, dans une première étape, avec un ester d'acide cyanoacétique de formule générale 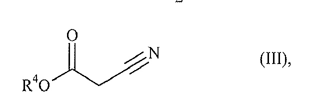 dans laquelle R⁴ représente un groupe alkyle en C₁₋₁₀, pour donner lieu à un amide d'acide cyanoacétique de formule générale 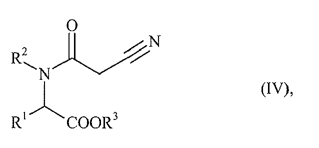 dans laquelle R¹, R² et R³ présentent les significations mentionnées ci-dessus, ou un sel correspondant, qui est transformé, dans une deuxième étape, en le composé cible (I) ou un sel correspondant par hydrogénation catalytique.

2. Procédé selon la revendication 1, **caractérisé en ce que** R¹ représente un groupe imidazol-4-ylméthyle qui peut être éventuellement substitué comme indiqué dans la revendication 1 pour R¹, et R² représente un atome d'hydrogène.

3. Procédé selon la revendication 1 ou 2, **caractérisé en ce que** l'on utilise de l'éthanol et/ou du *N*,*N*-diméthylformamide en tant que solvant dans la première étape.

4. Procédé selon l'une quelconque des revendications 1 à 3, **caractérisé en ce que** l'on utilise, en tant que catalyseur dans la deuxième étape, un catalyseur parmi le groupe constitué du rhodium sur du charbon actif, du rhodium sur de l'oxyde d'aluminium, du nickel de Raney et du cobalt de Raney.

5. Amides d'acide cyanoacétique de formule générale 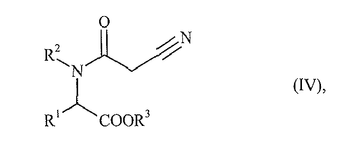 dans laquelle R¹ et R² présentent la signification mentionnée dans la revendication 2 et R³ est tel que défini selon la revendication 1, ainsi que leurs sels.

6. *N*^{α}-(Cyanoacétyl)-L-histidine et ses esters de formule 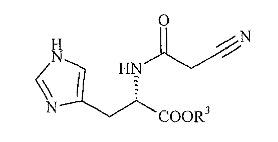 dans laquelle R³ présente la signification mentionnée dans la revendication 1, ainsi que leurs sels et leurs tautomères.

7. N^{α}-(Cyanoacétyl)-3-méthyl-L-histidine et ses esters de formule 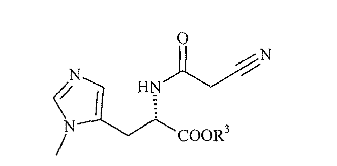 dans laquelle R³ présente la signification mentionnée dans la revendication 1, ainsi que leurs sels.
